# EUROPEAN PATENT SPECIFICATION

(11) **EP 0 295 859 B1**
(45) Date of publication and mention of the grant of the patent: **17.11.1994**
(21) Application number: 88305404.1
(22) Date of filing: 14.06.1988
(51) Int. Cl.: C07K 3/08, C12P 21/02, C12N 15/18

(54) **Production of proteins in active forms**
Herstellung von Proteinen in aktiver Form
Préparation de protéines dans les formes actives

(30) Priority: 15.06.1987 AU 2472/87
(43) Date of publication of application: 21.12.1988
(73) Proprietor: SOUTHERN CROSS BIOTECH PTY.LTD., Dandenong, Victoria 3175 (AU)
(72) Inventor: Patroni Joseph John, Preston West, Victoria (AU); Brandon Malcolm Roy, Ivanhoe East Victoria (AU)
(74) Representative: Harding, Richard Patrick

(56) References cited:
- EP-A- 226 448
- GB-A- 2 001 326
- US-A- 4 462 940
- US-A- 4 992 531
- METHODS IN ENZYMOLOGY, vol. 104, pages 305-318, Academic Press, Inc.; L.M.HJELMELAND et al.: "Solubilization of functional membrane proteins"
- BICHEMICAL J., vol. 240, 1986, pages 1-12, GB; F.A.O. MARSTON: "Thepurification of eukaryotic polypeptides synthesized in Escherichia coli"
- Anal.Biochem. Vol.145, 170-176 (1985)
- Biochem.J. Vol.285, 871-879 (1992)

## Description

The present invention relates to a method for the preparation of a protein in a biologically active or native form.

Recombinant DNA technology provides potentially extremely valuable means of synthesizing amounts of desirable eukaryotic (usually mammalian) proteins such as hormones, interferons, and enzymes. Although it has proved to be relatively easy to manipulate organisms, such as bacteria to produce the desired protein, the host organism may not secrete the over-produced protein product into the culture medium. Thus physical or chemical lysis of the organisms (for example bacteria), followed by mechanical isolation of the insoluble desired protein is usually necessary. In the prior art solubilisation of the insoluble protein then proceeds with high concentrations of denaturants such as an aqueous urea or guanidine hydrochloride (International Patent Application WO 83/04418). Thus solubilisation has been conducted with relatively pure forms of the desired protein obtained by a multistep process. Such processes are capital intensive and are best avoided when applied industrially.

US-A-4462940 discloses a process for recovering and purifying human B-interferon-like polypeptides from transformed bacterial cells. These cells are concentrated and disrupted and the resulting cell paste is solubilised using the anionic surfactant SDS.

Hjelmeland, L. M. and Chrambach, A., "Solubilisation of functional membrane proteins", Methods in Enzymology, Vol. 104 p. 305-318 describes methods for the solubilisation of functional membrane proteins. Detergents useful for the solubilisation of membrane proteins are discussed, together with the factors influencing the choice of detergent. The use of cationic surfactants for solubilisation of membrane proteins is not disclosed.

GB-A-2001326 discloses a process for the isolation of glycoproteins from paramyxovirus by treating the virus with a cationic detergent to selectively solubilise the glycoprotein components. The glycoprotein components are separated from residual intact sub-viral particles using conventional methods, and may then be used in vaccines.

In EP-A-226448 the Applicants have described a highly advantageous, albeit multistep economical method for the recovery of proteins in a soluble form from an insoluble protein source utilising a cationic surfactant. Whilst this process allows for the efficient recovery of proteins in a soluble form, the ultimate recovery of active protein has been limited. For example, the overall recovery is normally less than 50%. Significant losses may occur in the collection of host cells, their lysis and concentration of protein aggregates thus released via physical concentrative methods including differential centrifugation.

Accordingly, it is an object of the present invention to overcome, or at least alleviate, one or more of the difficulties related to this prior method.

According to the present invention there is provided a method for the recovery of protein in a solubilised form from host cells comprising:
providing host cells incorporating an insoluble synthesized or expressed protein;
providing at least one cationic surfactant; and
treating the host cells with said at least one cationic surfactant in an amount sufficient to effect lysis of the host cell and subsequent solubilisation of the insoluble protein to form a solubilised protein, wherein said cationic surfactant includes a cation selected from the group consisting of cetyl trimethylammonium cations, cetyl pyridinium cations, tetradecyl trimethylammonium cations, dodecyl trimethylammonium cations, mixed n-alkyl dimethyl benzyl ammonium cations and N,N-dimethyl-N-[2-[2-[4-(1,1,3,3,-tetramethylbutyl)phenoxy]ethoxy]ethyl]benzenemethanaminium cations.

Preferably the method for recovery of proteins includes providing a fermentation broth including host cells incorporating a synthesized or expressed protein and isolating the host cells therefrom. The isolation step may be undertaken utilising any suitable method. Flotation, centrifugation, filtration or ultrafiltration may be used.

The host cell, in a preferred aspect, may be pretreated to kill the cell or weaken the cell membrane to facilitate lysis.

The product solution may be purified utilising either differential centrifugation, differential precipitation, chromatography or filtration. This will remove impurities such as insoluble unwanted contaminants, unwanted cell debris and other unwanted macromolecules.

Desirably the amount of the at least one cationic surfactant exceeds the critical micelle concentration.

The present invention is particularly applicable to the solubilisation and recovery of biologically important proteins synthesized by microorganisms and eukaryotic cell lines which have been modified by recombinant DNA technology. The desired protein may comprise an inclusion body in a host cell which may have been transformed or transfected with a vector including a gene coding for the protein.

The protein which may be recovered according to the present invention may be selected from monomeric and polymeric intracellular proteins such as those found in cellular inclusion bodies and cytoplasmic aggregates. The inclusion bodies may be selected from biologically important polypeptides and peptides including growth hormones porcine, ovine and bovine, interferons, immunogens and lymphokines, or heterogeneous or homogeneous polymers thereof.

Preferably the at least one cationic surfactant is present in amount of from approximately 2.5 to 50% weight/volume, more specifically 2.5 to 20% weight/volume. The upper limit of surfactant content may vary and be limited by the solubility of the selected surfactant.

We have found that it is possible to solubilise aggregates of desired proteins produced and contained within whole cells, including inclusion bodies, by treatment of the cell and its components with a cationic surfactant in water, either in the presence or absence of a polar organic solvent. The process can be rapid (5-60 min), and recovery of the solubilised protein can be optimally effected very easily. Only small quantities of inexpensive reagents, which are readily available and recyclable, are required. For example, the bulk of the solubilising agent may be water.

The method of the invention may also be applied to a method for the recovery of cellular proteins which method includes providing host cells incorporating the desired protein, at least one cationic surfactant and at least one polar organic solvent; treating the cell with a mixture of from approximately 5 to 70% volume/volume of the at least one polar organic solvent and at least one cationic surfactant in an amount sufficient to effect cell lysis and protein solubilisation without substantial modification to the native conformation of the protein; and separating the solubilised protein from the resulting mixture.

The protein may be maintained in an aqueous solution comprising a polar organic solvent and suitable buffering salts. Preferably the pH of the solution is optimised to ensure protein solubility and stability of the solution. The presence of a polar organic solvent, such as acetonitrile or acetic acid, preferably at a concentration of 5 to 70% alters the interaction between the insoluble protein and the aqueous solvent, thereby facilitating the solubility of the hydrophobic regions of the protein. More preferably the concentration of the organic solvent is 10 to 20%.

The incorporation of a cationic surfactant, such as a quaternary ammonium compound, at a level exceeding the critical micelle concentration and sufficient to overcome the associative forces of the cell wall and those within the protein aggregate, promotes lysis of the cell and the segregation, disruption and solubilisation of the inclusion body constituents.

The method may be conducted at any suitable temperature above the freezing point of the solution. A temperature in the range of approximately 4 to 25°C is preferred.

The addition of an aqueous solution of a suitable surfactant to a dried powder of an aqueous slurry of the host cells is also a desirable and efficient means of solubilisation of cellular proteins. The addition of the at least one cationic surfactant is desirably at levels above the critical micelle concentration and within the limit of its solubility and economy.

In contrast to some prior art, the proteins may be solubilised in a mild near neutral environment. Furthermore, only low concentrations of the solubilising agents are required and these may be readily removed. Due to the chemical nature of the solubilising agent, the recovery method is compatible with later processing steps, in contradistinction to the severe solubilisation treatments of purified inclusion bodies in the prior art. The solubilising agent has been found to be compatible with other ingredients encountered in processing of protein aggregates. For example dithiothreitol, mercaptoethanol, glutathione, cysteine, cystine, dimethylsulfone, urea, thiourea, sodium and potassium hydroxides, borates or mineral acids.

The scope of the invention comprehends the use of all suitable single and multiple chain nitrogen or phosphorous cationic surfactants with various head groups, counter ions and branched or derivatised carbon chains.

Where the term halide appears, it should be understood, that the selection of the halide ions is illustrative only; the identity of the anion in this case is uncritical. For example the halide may be substituted by other anions, e.g. sulfonates e.g. p-toluene sulfonates.

More preferably the cationic surfactant is cetyl trimethylammonium bromide or cetyl pyridinium chloride.

It is preferred that the cationic surfactant selected is one which does not absorb in the region of the ultraviolet spectrum where polypeptide absorbance is maximal, e.g. cetyl trimethylammonium bromide.

The invention provides significant economic advantages in large scale purification systems.

The liquor product so formed includes the desired protein in a soluble form. Where the impurity levels are higher than required, standard purification procedures may be used. Chromatographic procedures are particularly useful.

The method according to the present invention may include the further step of separating the solubilised protein from the resulting crude solution.

The separation step may include differential elution of the solubilised protein through a chromatographic column, dialysis, ultrafiltration, differential precipitation, or ligand specific isolation. The chromatographic column may be a high performance liquid chromatography (HPLC) column, optionally a reversed phase high performance liquid chromatography (RP-HPLC) column. A column sold under the trade designation TSK-GEL (LC) and available from Toyo Soda Manufacturing Co. Ltd. (Japan) or Ultrapore RPSC and available from (Beckman Instruments Inc. (California, United States of America) have been found to be suitable. Due to the nature of the solubilising agent, the separation step may be conducted utilizing other known forms of chromatography including chromatography of the molecular sieve type, e.g. gel filtration chromatography, or ion exchange chromatography, hydrophobic interaction chromatography, and ligand specific chromatography. Preferably the chromatography eluant is an aqueous solution of a cationic, anionic or zwitterionic surfactant. A dilute solution may be used. The cationic, anionic or zwitterionic surfactant may be present in amounts of from approximately 0.25% weight/volume to approximately 2.0% weight/volume, more preferably 0.4% weight/volume.

It will be understood that the chromatographic separation also functions to purify the protein product.

It will be understood that the method according to the present invention may be utilised in a method for the analysis of a polypeptide sample wherein the sample to be tested is subjected to the recovery process thereof. The results may provide a quantitative analysis of the composition of the polypeptide sample.

Embodiments of the present invention will now be illustrated by way of example only with reference to the following non-limiting examples, and the accompanying figures.

### EXAMPLE 1

An experiment was conducted with a fermentation liquor including transformed E.coli cells incorporating inclusion bodies. The E.coli cells contained 1-190AA methionine-porcine growth hormone derived from plasmid pMG939. The cells were concentrated utilising ultrafiltration techniques washed twice with an aqueous solution of Triton X-100 (0.5%) and EDTA (10 mM) and twice with aqueous EDTA (5 mM).

The cells were treated with aqueous cetyl trimethylammonium bromide (20% w/v) which treatment also effected lysis of the cells. The treatment was conducted in a test tube, and the mixture agitated for 1 hour at room temperature. The mixture was centrifuged (13000 r.p.m., 10 min) on a Beckman Microfuge II to give a clear supernatant and an insoluble pellet. A small portion of the pellet was fixed and embedded into L.R. White resin and the block section sectioned for comparison by electron microscopy with the untreated material.

The results, in marked contrast to the untreated material, showed that no inclusion bodies were to be seen after the solubilisation procedure.

### EXAMPLE 2

An experiment was performed with transformed E.coli cells containing the expressed variant 4-190AA porcine growth hormone derived from plasmid pMG936. The cells were collected from the fermenter and concentrated by centrifugation (9,000g, 5 minutes). A portion of the wet cell pellet (50mg) was vigorously agitated (1h) with N-llauroyl-N-methyltaurine sodium salt (3ml of 12% w/v) and dithiothreitol (3% w/v) in 0.1M TRIZMA (pH 10.0), 0.01 M EDTA. The mixture was then clarified by centrifugation (50,000 g; 10 minutes). An immuno-dot blot analysis of the clear supernatant using nitro-cellulose paper and a monoclonal antibody to porcine growth hormone confirmed the presence and solubilisation into solution of the expressed growth hormone initially contained within the cell. This example was not in accordance with the invention.

### EXAMPLE 3

An example was performed with E.coli cells incorporating inclusion bodies comprising 1-190AA methionine-porcine growth hormone derived from plasmid pMG939. The cells were isolated from the fermenter broth by centrifugation (9,000 g, 5 minutes). A portion of the wet pellet (50 mg) was then subjected to vigorous agitation (1 h) with an aqueous solution of one of the surfactants listed below (3.0 ml of 10 - 20% in 0.1M TRIZMA, pH 10.0) in a test tube at 25°C and in the presence of -mercaptoethanol 2% (v/v). As with previous experiments substantial solubilisation of the inclusion bodies contained initially within the cells had taken place.
(a) cetyl-pyridinium chloride
(b) tetradecyltrimethylammonium bromide
(c) dodecyltrimethylammonium bromide
(d) mixed n-alkyl dimethyl benzyl ammonium chloride (50% C-14, 40% C-12, 10% C-16)
(e) N,N-dimethyl-N-[2-[2-[4-(1,1,3,3-tetramethylbutyl) phenoxy]ethoxy]ethyl] benzenemethanaminium chloride.

## Claims

1. A method for the recovery of protein in a solubilised form from host cells comprising:
providing host cells incorporating an insoluble synthesized or expressed protein;
providing at least one cationic surfactant; and
treating the host cells with said at least one cationic surfactant in an amount sufficient to effect lysis of the host cell and subsequent solubilisation of the insoluble protein to form a solubilised protein, wherein said cationic surfactant includes a cation selected from the group consisting of cetyl trimethylammonium cations, cetyl pyridinium cations, tetradecyl trimethylammonium cations, dodecyl trimethylammonium cations, mixed n-alkyl dimethyl benzyl ammonium cations and N,N-dimethyl-N-[2-[2-[4-(1,1,3,3,-tetramethylbutyl)phenoxy]ethoxy]ethyl]benzenemethanaminium cations.

2. A method according to claim 1, wherein said insoluble synthesized or expressed protein is selected from the group consisting of growth hormones, interferons, immunogens and lymphokines.

3. A method according to claim 1 or 2, wherein said at least one cationic surfactant is present in an amount of approximately 2.5-50% weight for volume.

4. A method according to Claim 1, 2 or 3, wherein said cationic surfactant is cetyl trimethylammonium bromide.

5. A method according to any preceding claim, wherein said at least one cationic surfactant is present in an amount exceeding the micelle concentration.

6. A method according to any preceding claim, further comprising the step of separating the solubilised protein, wherein the separation step is selected from the group consisting of differential elution of the solubilised protein through a chromatographic column, dialysis, ultrafiltration and differential precipitation.

## Patentansprüche

1. Verfahren zur Gewinnung von Protein in löslicher Form von Wirtszellen,
das unlösliches, durch Synthese oder Expression hergestelltes Protein enthaltende Wirtszellen vorsieht,
das wenigstens ein kationisches Tensid vorsieht, und bei dem die Wirtszellen mit besagtem wenigstens einem kationischen Tensid behandelt werden, in einer Menge, die ausreichend ist, um die Lyse der Wirtszelle und die anschliessende Solubilisierung des unlöslichen Proteins zur Bildung eines löslichen Proteins zu bewirken, dadurch gekennzeichnet, daß das besagte kationische Tensid ein Kation ausgewählt aus der Gruppe der Cetyl-Trimethylammonium-Katione, Cetylpyridinium-Katione, Tetradecyl-Trimethylammonium-Katione, Dodecyl-Trimethylammonium-Katione, der gemischten n-Alkyl-Dimethyl-Benzyl-Ammoniumkatione sowie der N,N-Dimethyl-N-[2-[2-[4-(1,1,3,3,-Tetramethylbutyl)-Phenoxy]Ethoxy]Ethyl]Benzolmethanaminium-Katione enthält.

2. Verfahren nach Anspruch 1, dadurch gekennzeichnet, daß das unlösliche, durch Synthese oder Expression hergestellte Protein ausgewählt ist aus der Gruppe der Wachstumshormone, Interferone, Immunogene und Lymphokine.

3. Verfahren nach Anspruch 1 oder 2, dadurch gekennzeichnet, daß besagtes wenigstens ein kationisches Tensid in einer Menge von ungefähr 2,5-50% Gewicht/Volumen vorhanden ist.

4. Verfahren nach Anspruch 1, 2 oder 3, dadurch gekennzeichnet, daß das besagte kationische Tensid Cetyl-Trimethylammoniumbromid ist.

5. Verfahren nach einem der vorstehenden Ansprüche, dadurch gekennzeichnet, daß besagtes wenigstens ein kationisches Tensid in einer die Mizellerkonzentration übersteigenden Menge vorhanden ist.

6. Verfahren nach einem der vorstehenden Ansprüche, das die Trennung des solubilisierten Proteins umfasst, dadurch gekennzeichnet, daß die Trennung ausgewählt ist aus der Gruppe bestehend aus Differentialelution des solubilisierten Proteins durch eine chromatographische Säule, Dialyse, Ultrafiltration und Differentialtrennung.

## Revendications

1. Procédé pour récupérer une protéine sous une forme solubilisée à partir de cellules hôtes comprenant :
- la fourniture de cellules hôtes incorporant une protéine insoluble produite par synthèse ou expression,
- la fourniture d'au moins un agent tensio-actif cationique ; et le traitement des cellules hôtes avec ledit au moins un tensio-actif cationique en une quantité suffisante pour effectuer la lyse de la cellule hôte et solubilisation ultérieure de la protéine insoluble pour former une protéine solubilisée, caractérisé en ce que le tensio-actif cationique comprend un cation choisi parmi le groupe consistant en cations de triméthylammonium, cations de cétyl pyridinium, cations de tétradécyl triméthylammonium, cations de dodécyl triméthylammonium, cations de n-alkyl diméthyl benzyle ammonium mixtes et cations de N,N-di-méthyl-N-[2-[2-[4-(1,1,3,3,-tétraméthylbutyl)phénoxy]-éthoxy]éthyl] benzèneméthanaminium.

2. Procédé selon la revendication 1, caractérisé en ce que ladite protéine insoluble produite par synthèse ou expression est choisie parmi le groupe constitant en normones de croissance, interférons, immunogènes et lymphokines.

3. Procédé selon la revendication 1 ou 2, caractérisé en ce que au moins un tensio-actif cationique est présent en une quantité d'environ 2,5-50 % en poids/volume.

4. Procédé selon la revendication 1, 2 ou 3, caractérisé en ce que ledit tensio-actif cationique est le bromure de cétyl triméthylammonium.

5. Procédé selon l'une quelconque des revendications précédentes, caractérisé en ce que ledit au moins un tensio-actif cationique est présent en une quantité excédant la concentration micellaire.

6. Procédé selon l'une quelconque des revendications précédentes, comprenant en outre l'étape de séparation de la protéine solubilisée, caractérisé en ce que l'étape de séparation est choisie parmi le groupe consistant en élution différentielle de la protéine solubilisée sur une colonne de chromatographie dialyse, ultra-filtrations et précipitation différentielle.
